# EUROPEAN PATENT APPLICATION

(11) **EP 1 813 605 A1**
(43) Date of publication of application: **01.08.2007**
(21) Application number: 05800339.3
(22) Date of filing: 07.11.2005
(51) Int. Cl.: C07D 257/04

(54) **METHOD FOR PREPARING 2-ALKOXY-5-TETRAZOLYL-BENZALDEHYDE COMPOUND**

(30) Priority: 11.11.2004 JP 2004327929
(71) Applicant: TOYO KASEI KOGYO COMPANY LIMITED, Osaka-shi, Osaka 530-0004 (JP)
(72) Inventor: HAGIYA, Kazutake, c/o Chemicals Research Center of, Takasago-shi, Hyogo 676-0082 (JP)
(74) Representative: Müller-Boré & Partner Patentanwälte
(86) International application number: PCT/JP2005/020364
(87) International publication number: WO 2006/051751

(57) **Abstract**

The present invention provides a method for safely and efficiently preparing 2-alkoxy-5-tetrazolyl-benzaldehyde compounds by formylating (4-alkoxyphenyl)-tetrazole compounds.

The present invention provides a method for preparing 2-alkoxy-5-(alkyltetrazole-5-yl)-benzaldehyde by reacting a 5-(4-alkoxyphenyl)-alkyl-tetrazole with hexamethylenetetramine in a sulfonic acid solvent, followed by hydrolysis; and a method for preparing 2-alkoxy-5-(tetrazole-2-yl)-benzaldehyde compounds by reacting 2-(4-alkoxyphenyl)-2H-tetrazole compounds with hexamethylenetetramine in a sulfonic acid solvent, followed by hydrolysis.

## Description

### TECHNICAL FIELD

The present invention relates to methods for preparing 2-alkoxy-5-tetrazolyl-benzaldehyde compounds.

### BACKGROUND ART

2-Alkoxy-5-tetrazolyl-benzaldehyde compounds have been used as intermediates for pharmaceutical compositions used in treating symptoms (excluding vomiting) transmitted by tachykinin, and known to be useful as pharmaceutical intermediates (JP11-106341A).

Formylation reactions for aromatic compounds have been researched for a long period of time, and there are a variety of reported processes. Typical examples are (1) a process that uses dimethylformamide and phosphorus oxychloride (Org. Synth. Coll. Vol. 4, 1963, p. 539); (2) a process that uses dimethylformamide and trifluoromethanesulfonic anhydride (J. Chem. Soc., Chem. Commun., 1990, p. 1571); (3) a process that uses hexamethylenetetramine and trifluoroacetic acid (J. Org. Chem., vol. 37, 1972, p. 3972); (4) a process that uses imidazole and trifluoroacetic anhydride (Tetrahedron, vol. 36, 1980, p. 2505); (5) a process that uses carbon monoxide, hydrochloric acid and aluminium (III) chloride (Org. React., vol. 5, 1960, p. 290); and (6) a process that uses zinc (II) cyanide, hydrochloric acid and aluminium (III) chloride (Chem. Rev., vol. 63, 1963, p. 526).

However, when (4-alkoxyphenyl)-tetrazole compounds are subjected to formylation reactions according to processes (1) to (4) above, the reactions hardly progress. Processes (5) and (6) may be hazardous when used for commercial production because in process (5) toxic carbon monoxide is used, and in process (6) toxic hydrogen cyanide is generated in the reaction system.

Therefore, formylation reactions for (4-alkoxyphenyl)-tetrazole compounds do not progress or are hazardous when prior-art processes are used. Prior-art processes are thus not industrially advantageous.

### DISCLOSURE OF INVENTION

### PROBLEMS TO BE SOLVED BY THE INVENTION

An object of the present invention is to provide a method for preparing a 2-alkoxy-5-tetrazolyl-benzaldehyde compound in a safe and efficient manner by formylating a (4-alkoxyphenyl)-tetrazole compound.

### MEANS FOR SOLVING THE PROBLEMS

The present inventors conducted extensive research and found that 2-alkoxy-5-tetrazolyl-benzaldehyde compounds can be safely and efficiently prepared by subjecting a (4-alkoxyphenyl)-tetrazole compound to reaction with hexamethylenetetramine in a sulfonic acid solvent, and then to hydrolysis. The present invention has been accomplished based on this finding.

In particular, the present invention provides methods for preparing 2-alkoxy-5-tetrazolyl-benzaldehyde compounds as described below.
1. A method for preparing a 2-alkoxy-5-(alkyltetrazole-5-yl)-benzaldehyde represented by Formula (II); 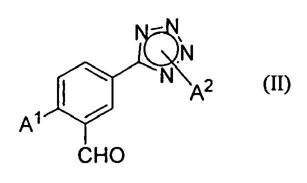 wherein A¹ is an alkoxyl group, and A² is an alkyl group,
   the method comprising reacting a 5-(4-alkoxyphenyl)-alkyl-tetrazole represented by Formula (I); 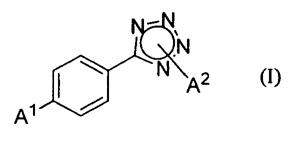 wherein A¹ and A² are as defined above, with hexamethylenetetramine in a sulfonic acid solvent, followed by hydrolysis.
2. The method according to Item 1, wherein the 5-(4-alkoxyphenyl)-alkyl-tetrazole is 5-(4-methoxyphenyl)-1-methyl-1H-tetrazole or 5-(4-methoxyphenyl)-2-methyl-2H-tetrazole.
3. The method according to Item 1 or 2, wherein the sulfonic acid solvent is a mixed solvent of methanesulfonic acid and trifluoromethanesulfonic acid.
4. The method according to any one of Item 1 to 3, wherein hexamethylenetetramine is used in an amount of 1.0 to 3.0 mol per mol of the 5-(4-alkoxyphenyl)-alkyl-tetrazole.
5. A method for preparing a 2-alkoxy-5-(tetrazole-2-yl)-benzaldehyde compound represented by Formula (IV); 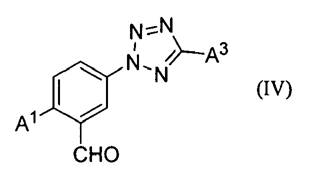 wherein A¹ is an alkoxyl group and A³ is a hydrogen atom or an alkyl group,
   the method comprising reacting a 2-(4-alkoxyphenyl)-2H-tetrazole compound represented by Formula (III); 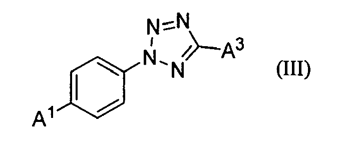 wherein A¹ and A³ are as defined above, with hexamethylenetetramine in a sulfonic acid solvent, followed by hydrolysis.
6. The method according to Item 5, wherein the 2-(4-alkoxyphenyl)-2H-tetrazole compound is 2-(4-methoxyphenyl)-2H-tetrazole or 2-(4-methoxyphenyl)-5-methyl-2H-tetrazole.
7. The method according to Item 5 or 6, wherein the sulfonic acid solvent is a mixed solvent of methanesulfonic acid and trifluoromethanesulfonic acid.
8. The method according to any one of Items 5 to 7, wherein hexamethylenetetramine is used in an amount of 1.0 to 3.0 mol per mol of the 2-(4-alkoxyphenyl)-2H-tetrazole compound.

The present invention is explained in detail below.

A¹, A² and A³ in Formulae (I) to (IV) are described first.

The alkyl moiety of an alkoxyl group represented by A¹ may be linear or branched. When branched, the number and position(s) of branch(es) are not limited. For the reaction to progress smoothly, the alkyl moiety preferably has 1 to 10 carbon atoms, and more preferably 1 to 4 carbon atoms. Preferable and specific examples of alkoxyl groups are methoxy, ethoxy, *n-*propoxy, isopropoxy, *n*-butoxy, but-2-oxy, 2-methylprop-l-oxy, 2-methylprop-2-oxy and like groups.

An alkyl group represented by A² and A³ may be independently linear or branched. When branched, the number and position(s) of branch(es) are not limited. For the reaction to progress smoothly, the alkyl group preferably has 1 to 10 carbon atoms, and more preferably 1 to 4 carbon atoms. Preferable and specific examples are methyl, ethyl, *n*-propyl, isopropyl, *n*-butyl, sec-butyl, isobutyl, tert-butyl and like groups.

In the present invention, a methoxy group is particularly preferable for A¹. A methyl group is particularly preferable for A². A hydrogen atom or a methyl group is particularly preferable for A³.

The 5-(4-alkoxyphenyl)-alkyl-tetrazole represented by Formula (I) may be prepared according to any process. Specific preferable examples are 5-(4-methoxyphenyl)-1-methyl-1H-tetrazole and 5-(4-methoxyphenyl)-2-methyl-2H-tetrazole.

The 2-(4-alkoxyphenyl)-2H-tetrazole compound represented by Formula (III) may be prepared according to any process. Specific preferable examples are 2-(4-methoxyphenyl)-2H-tetrazole and 2-(4-methoxyphenyl)-5-methyl-2H-tetrazole.

The amount of hexamethylenetetramine used in preparing the 2-alkoxy-5-(alkyltetrazole-5-yl)-benzaldehyde of Formula (II) in the present invention is preferably 1.0 to 3.0 mol, and more preferably 1.2 to 2.0 mol, per mol of the 5-(4-alkoxyphenyl)-alkyl-tetrazole of Formula (I).

The amount of hexamethylenetetramine used in preparing the 2-alkoxy-5-(tetrazole-2-yl)-benzaldehyde compound of Formula (IV) in the present invention is preferably 1.0 to 3.0 mol, and more preferably 1.2 to 2.0 mol, per mol of the 2-(4-alkoxyphenyl)-2H-tetrazole compound of Formula (III).

Sulfonic acid solvents usable in the present invention are not limited insofar as water is not contained therein. Preferable sulfonic acid solvents are those that can dissolve the 5-(4-alkoxyphenyl)-alkyl-tetrazole of Formula (I) or the 2-(4-alkoxyphenyl)-2H-tetrazole compound of Formula (III). Specific examples are methanesulfonic acid, ethanesulfonic acid, trifluoromethanesulfonic acid, pentafluoroethanesulfonic acid, etc. Such solvents may be used either singly as homosolvents or in suitable combinations as mixed solvents. Among such sulfonic acid solvents, particularly preferable is a mixed solvent in which the ratio of methanesulfonic acid to trifluoromethanesulfonic acid is 1 : 0.6 to 1.5 (volume ratio). The amount of sulfonic acid solvent is preferably 1 to 15 ml, and more preferably 5 to 10 ml, per gram of the 5-(4-alkoxyphenyl)-alkyl-tetrazole of Formula (I) or the 2-(4-alkoxyphenyl)-2H-tetrazole compound of Formula (III).

The reaction of hexamethylenetetramine with the 5-(4-alkoxyphenyl)-alkyl-tetrazole of Formula (I) or the 2-(4-alkoxyphenyl)-2H-tetrazole compound of Formula (III) can be conducted in a sulfonic acid solvent with heating. Excessively low reaction temperatures slow the reaction, and excessively high reaction temperatures result in the generation of large amounts of by-products. Therefore, the reaction temperature is preferably about 50 to about 150°C, and more preferably about 80 to about 100°C. The reaction time is preferably about 1 to about 8 hours, and more preferably about 2 to about 5 hours.

After reaction, the reaction system is cooled to room temperature, and then either water is introduced into the reaction system or the reaction solution is introduced into water for hydrolysis. The amount of water used is preferably 1 ml to 30 ml and more preferably 5 ml to 20 ml, per gram of the 5-(4-alkoxyphenyl)-alkyl-tetrazole of Formula (I) or the 2-(4-alkoxyphenyl)-2H-tetrazole compound of Formula (III). The temperature for hydrolysis is preferably about 0 to about 30°C. The hydrolyzation time is preferably about 15 minutes to about 2 hours, and more preferably about 30 minutes to about 1 hour.

Subsequently, extraction, separation, drying, solvent evaporation or like ordinary procedures are performed to obtain a crude product. Purification by crystallization, recrystallization, column chromatography, or the like can then be performed to obtain the 2-alkoxy-5-(alkyltetrazole-5-yl)-benzaldehyde of Formula (II) or the 2-alkoxy-5-(tetrazole-2-yl)-benzaldehyde compound of Formula (IV).

### EFFECTS OF THE INVENTION

The present invention makes it possible to prepare 2-alkoxy-5-tetrazolyl-benzaldehyde compounds in a safe and efficient manner by formylating (4-alkoxyphenyl)-tetrazole compounds.

### BEST MODE FOR CARRYING OUT THE INVENTION

Examples are given below to illustrate the invention in more detail.

### Example 1

2-Methoxy-5-(1-methyl-1H-tetrazole-5-yl)-benzaldehyde

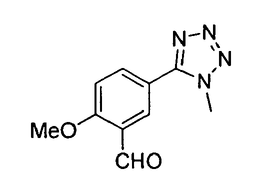

Into a 10 ml flask were introduced 150 mg (0.79 mmol) of 5-(4-methoxyphenyl)-1-methyl-1H-tetrazole, 0.75 ml of methanesulfonic acid, 0.75 ml of trifluoromethanesulfonic acid and 222 mg (1.58 mmol) of hexamethylenetetramine. The mixture was heated to 100°C and allowed to react for 3 hours. After completion of the reaction, the resultant reaction mixture was cooled to room temperature, and introduced into 2 ml of water cooled in an ice bath, followed by agitation at 20°C for 30 minutes. Subsequently, the reaction solution was extracted with methylene chloride (3 ml x 3) and washed with 5 ml of water. The solvent contained therein was distilled off by vacuum concentration. The thus-obtained crude product was purified using a column chromatography (using silica gel and methylene chloride), giving 86 mg of white solid 2-methoxy-5-(1-methyl-1H-tetrazole-5-yl)-benzaldehyde (yield: 50%).
Melting point: 122.8 to 125.0°C
IR (KBr, cm⁻¹): 1682, 1614, 1584, 1481, 1449, 1406, 1395, 1379, 1283, 1184, 1016, 901, 822, 747, 706, 646
¹H-NMR (CDCl₃) : δ = 10.50 (s, 1H), 8.13 (d, J = 2.4 Hz, 1H), 8.11 (dd, J = 8.5 Hz, 2.4 Hz, 1H), 7.20 (d, J = 8.5 Hz, 1H), 4.19 (s, 3H), 4.03 (s, 3H)
Elemental analysis:
Value calculated for C₁₀H₁₀N₄O₂: C, 55.04%; H, 4.62%; N, 25.68%
Value found: C, 54.34%; H, 3.87%; N, 27.07%.

### Example 2

### 2-Methoxy-5-(2-methyl-2H-tetrazole-5-yl)-benzaldehyde

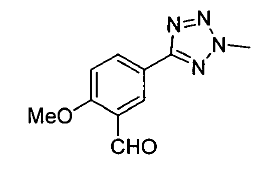

Into a 10 ml flask were introduced 150 mg (0.79 mmol) of 5-(4-methoxyphenyl)-2-methyl-2H-tetrazole, 0.75 ml of methanesulfonic acid, 0.75 ml of trifluoromethanesulfonic acid and 222 mg (1.58 mmol) of hexamethylenetetramine. The mixture was heated to 100°C and allowed to react for 3 hours. After completion of the reaction, the resultant reaction mixture was cooled to room temperature, and introduced into 2 ml of water cooled in an ice bath, followed by agitation at 20°C for 30 minutes. Subsequently, the reaction solution was extracted with methylene chloride (3 ml x 3) and washed with 5 ml of water. The solvent contained therein was distilled off by vacuum concentration. The thus-obtained crude product was purified using a column chromatography (using silica gel and methylene chloride), giving 103 mg of white solid 2-methoxy-5-(2-methyl-2H-tetrazole-5-yl)-benzaldehyde (yield: 60%).
Melting point: 138.7 to 141.3°C
IR (KBr, cm⁻¹): 1678, 1614, 1584, 1466, 1437, 1422, 1404, 1354, 1281, 1252, 1192, 1148, 1103, 1015, 849, 762, 725, 532
¹H-NMR (CDCl₃): δ = 10.49 (s, 1H), 8.57 (d, J = 2.2 Hz, 1H), 8.33 (dd, J = 8.8 Hz, 2.2 Hz, 1H), 7.10 (d, J = 8.8 Hz, 1H), 4.37 (s, 3H), 3.99 (s, 3H)
¹³C-NMR (CDCl₃): δ = 188.73, 164.01, 162.66, 133.83, 127.33, 124.95, 120.23, 112.09, 56.01, 39.58
Elemental analysis:
Value calculated for C₁₀H₁₀N₄O₂: C, 55.04%; H, 4.62%; N, 25.68%
Value found: C, 54.70%; H, 4.38%; N, 25.59%.

### Example 3

### 2-Methoxy-5-tetrazole-2-yl-benzaldehyde

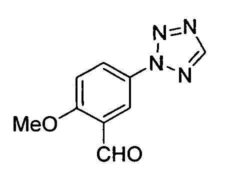

Into a 10 ml of flask were introduced 50 mg (0.26 mmol) of 2-(4-methoxyphenyl)-2H-tetrazole, 0.25 ml of methanesulfonic acid, 0.25 ml of trifluoromethanesulfonic acid and 74 mg (0.52 mmol) of hexamethylenetetramine. The mixture was heated to 100°C and allowed to react for 3 hours. After completion of the reaction, the resultant reaction mixture was cooled to room temperature, and introduced into 1 ml of water cooled in an ice bath, followed by agitation at 20°C for 30 minutes. Subsequently, the reaction solution was extracted with methylene chloride (2 ml x 3) and washed with 3 ml of water. The solvent contained therein was distilled off by vacuum concentration. The thus-obtained crude product was purified using a column chromatography (using silica gel and methylene chloride), giving 24 mg of white solid 2-methoxy-5-tetrazole-2-yl-benzaldehyde (yield: 42%).
¹H-NMR (CDCl₃): δ = 10.47 (s, 1H), 8.92 (s, 1H), 8.06 (d, J = 2.1 Hz, 1H), 7.99 (dd, J = 9.0 Hz, 2.1 Hz, 1H), 7.20 (d, J = 9.0 Hz, 1H), 4.03 (s, 3H).

## Claims

1. A method for preparing a 2-alkoxy-5-(alkyltetrazole-5-yl)-benzaldehyde represented by Formula (II); 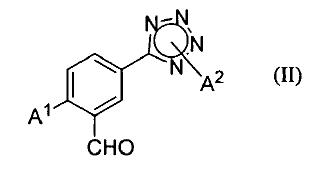 wherein A¹ is an alkoxyl group, and A² is an alkyl group,
the method comprising reacting a 5-(4-alkoxyphenyl)-alkyl-tetrazole represented by Formula (I); 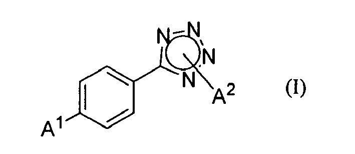 wherein A¹ and A² are as defined above, with hexamethylenetetramine in a sulfonic acid solvent, followed by hydrolysis.

2. The method according to Claim 1, wherein the 5-(4-alkoxyphenyl)-alkyl-tetrazole is 5-(4-methoxyphenyl)-1-methyl-1H-tetrazole or 5-(4-methoxyphenyl)-2-methyl-2H-tetrazole.

3. The method according to Claim 1, wherein the sulfonic acid solvent is a mixed solvent of methanesulfonic acid and trifluoromethanesulfonic acid.

4. The method according to Claim 1, wherein hexamethylenetetramine is used in an amount of 1.0 to 3.0 mol per mol of the 5-(4-alkoxyphenyl)-alkyl-tetrazole.

5. A method for preparing a 2-alkoxy-5-(tetrazole-2-yl)-benzaldehyde compound represented by Formula (IV); 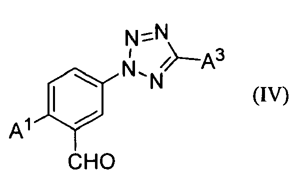 wherein A¹ is an alkoxyl group and A³ is a hydrogen atom or an alkyl group,
the method comprising reacting a 2-(4-alkoxyphenyl)-2H-tetrazole compound represented by Formula (III); 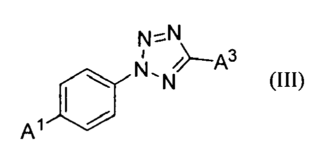 wherein A¹ and A³ are as defined above, with hexamethylenetetramine in a sulfonic acid solvent, followed by hydrolysis.

6. The method according to Claim 5, wherein the 2-(4-alkoxyphenyl)-2H-tetrazole compound is 2-(4-methoxyphenyl)-2H-tetrazole or 2-(4-methoxyphenyl)-5-methyl-2H-tetrazole.

7. The method according to Claim 5, wherein the sulfonic acid solvent is a mixed solvent of methanesulfonic acid and trifluoromethanesulfonic acid.

8. The method according to Claim 5, wherein hexamethylenetetramine is used in an amount of 1.0 to 3.0 mol per mol of the 2-(4-alkoxyphenyl)-2H-tetrazole compound.
